# EUROPEAN PATENT APPLICATION

(11) **EP 1 962 199 A2**
(43) Date of publication of application: **27.08.2008**
(21) Application number: 08250070.3
(22) Date of filing: 08.01.2008
(51) Int. Cl.: G06F 13/40

(54) **A communication apparatus**

(30) Priority: 08.01.2007 MY 0700019
(71) Applicant: O.Y.L. Research & Development Centre Sdn Bhd, 47000 Sungai Buloh, Selangor Darul Ehsan (MY)
(72) Inventor: Tan, Yee-Chyan c/o OYL R&D Centre Sdn. Bhd., 47000 Sungai Buloh, Selangor (MY); Toong, Ming Hong c/o OYL R&D Centre Sdn. Bhd., 47000 Sungai Buloh, Selangor (MY)
(74) Representative: Mounteney, Simon James

(57) **Abstract**

A communication apparatus for converting signal between differential interchange circuit and multiple devices having input and output terminals comprising: a timer for detecting logic level at idle state; a flip-flop having at least one output terminals for switching a signal from one to the other by means of an external signal; and at least one or more of logic gates for inverting said external signal from said circuit.

## Description

The present invention relates to a communication apparatus for connecting between multiple devices and a differential interchange circuit such as RS 485.

### Background Art

Interchange circuits such as RS 485 have signals being represented by a pair of lines as input/output terminals (A and B). These signals have different polarity and the receiver will need to determine the logic level by sensing the voltage difference between these terminals. This configuration has the advantage of attenuating the common mode noise since any induced noise or ground differentials will appear as common mode signals to the receiver.

However, when the terminals are connected wrongly, the signals at the receiver will be inverted as disclosed in CN 1649349A and US 5956523. The multiple devices system is unable to interpret the inverted signals received.

It is an object of the present invention to provide a connection between multiple devices and differential interchange circuits such as RS485.

### Summary of the Invention

In one aspect the invention is a communication apparatus for use with an interchange circuit having input and output terminals, said apparatus comprising:
a timer for detecting a logic level at an idle state;
a flip-flop having an output, the flip-flop changing logic states according to an external signal;
at least one logic gate for inverting said external signal.

The idle state may correspond to the idle state of the interchange circuit.

The logic level may be detected at a terminal of said interchange circuit.

The interchange circuit may have input and output terminals, and the communication apparatus may be adapted for detecting an error in the connection of an interchange circuit with multiple devices and adapted for converting a signal at input or output terminals of the interchange circuit.

The logic timer may be connected so as to detect said logic level at a receive terminal of said interchange circuit.

In another aspect the invention is a method for converting a signal between multiple devices and a differential interchange circuit, the method implemented by a communication apparatus, the method including:
receiving signals from the differential interchange circuit;
detecting a logic level at a receiving input terminal of the differential interchange circuit;
resetting via the logic level a timer in the communication apparatus and generating a pulse from an output of the timer;
triggering a flip-flop in the communication apparatus;
inverting the signals received; and
interpreting the received signals and establishing communication with the connected devices.

In a further aspect the invention is a communication apparatus for detecting the error in the signal and converting signal between differential interchange circuit and multiple devices having input and output terminals comprising:
a timer for resetting logic level;
a flip-flop having at least one output terminals for switching a signal from one to the other by means of an external signal; and
at lease one or more of logic gates for inverting said external signal from said circuit.

In a further aspect the invention is a method for converting signal between multiple devices and differential interchange circuit comprising:
receiving signals from differential interchange circuit;
detecting logic level at the receiving input/output terminals of the communication apparatus;
resetting the logic level by the timer in the communication apparatus and generating a pulse from the timer output in the communication apparatus;
triggering the flip-flop in the communication apparatus and inverting the signals received and
interpreting the received signals and establishing communication with the connected devices.

### Brief Description of the Drawings

The foregoing summary, as well as the following detailed description of preferred embodiments of the invention, will be better understood when read in conjunction with the appended drawings. For purpose of illustrating the invention, there are shown in the drawing embodiments, which are presently preferred. It should be understood, however, that the invention is not limited to the precise arrangements and instrumentalities shown.

Figure 1 shows a waveform if input/output terminals (A and B) are connected correctly of one embodiment of the present invention.

Figure 2 shows a waveform if input/output terminals (A and B) are connected wrongly of one embodiment of the present invention.

Figure 3 is a schematic diagram of the circuit connections of communication apparatus of one embodiment of the present invention;

Figure 4 shows waveforms at each respective pin when input/output terminals (A and B) are connected correctly with the connection of the communication apparatus of one embodiment of the present invention.

Figure 5 shows waveforms at each respective pin when input/output terminals (AA and B) are connected wrongly with the connection of the communication apparatus of one embodiment of the present invention.

### Detailed Description of the Preferred Embodiment

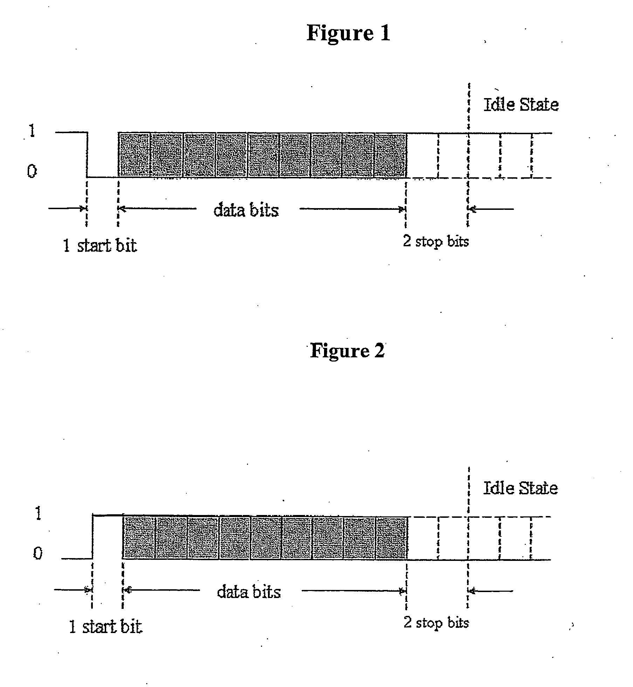

Figure 1 shows a waveform if input/output terminals (AB) are connected correctly of one embodiment of the present invention. Figure 2 shows a waveform if input/output terminals (A and B) are connected wrongly of one embodiment of the present invention. At idle state, the logic level at the receiving pin is high if input/output terminals (A and B) are connected correctly. If the input/output terminals (A and B) are wrongly connected the logic level at the receiving pin will be low at idle state. Without the auto-correcting circuitry in the communication apparatus of the present invention, multiple device are not able to interpret the signals received directly from the differential interchange circuit such as RS 485 and etc if the input/output terminals are wrongly connected.

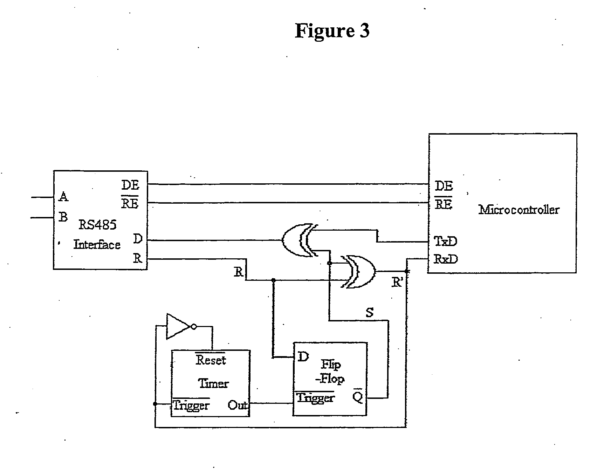

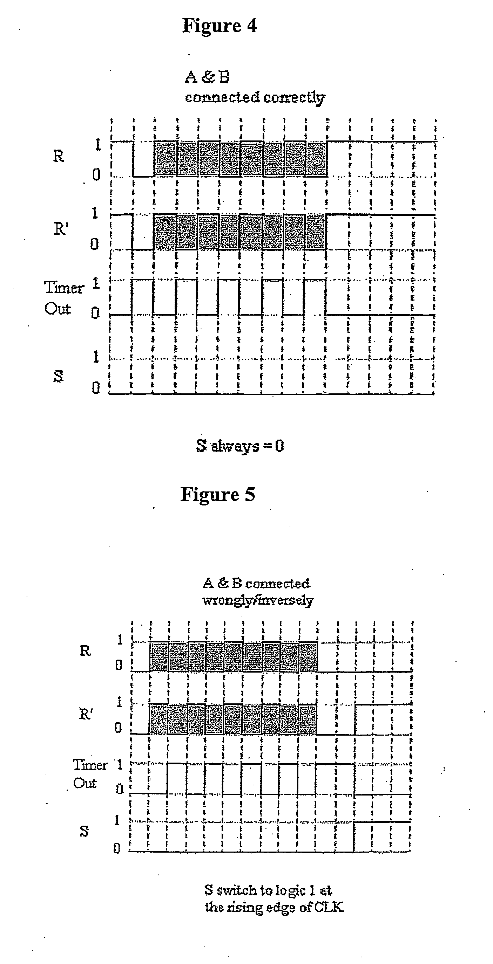

Figure 3 shows a schematic of the circuit connections of communication apparatus of one embodiment of the present invention. A communication apparatus of the present invention comprises a timer, a flip-flop and XOR logic gates, which are formed into a circuit. This circuit will detect the logic level at the receiving pin, R of the differential interchange circuit. If the input/output terminals (A and B) are correctly connected, the signal level will be logic high at idle state. The timer will always be reset in this case. When the timer resets, the negative going pulse from timer output triggers the flip-flop. The input D of the slip-flop is connected to R. In this case, R is always logic high when flip-flop is triggered. As a result, the output of the flip-flop will always be logic low as shown in Figure 4. The output of the flip-flop is connected to the input of the pair of XOR gates (S). The logic level of R and R' will be the same when logic level at S is low.

In the case that input/output terminals (A and B) are wrongly connected, the signal level will, be logic low at idle state. The timer will always reset at the presence of data signal but when line R returns to idle state, the timer will start counting. When the timer finishes counting, timer output becomes logic low and the negative going pulse at the timer output will trigger the flip-flop. When the flip-flop is triggered, the logic at R is low, causing the output of the flip-flop to become logic high. The output of the flip-flop is connected to the input of a pair of XOR gates (S). The logic level of R and R' will be inverted when logic level at S is high. Inverting the signal at R' makes the signal received by the microcontroller becomes logic high at idle state as shown in Figure 5. Therefore, the system is able to interpret the received signals and establish communications between devices.

Although the present invention has been described in relation to particular embodiments thereof, many other variations and modifications and other uses will become apparent to those skilled in the art. Therefore, the present invention should be limited not by the specific disclosure herein, but only by the appended claims.

## Claims

1. A communication apparatus for use with an interchange circuit having input and output terminals, said apparatus comprising:
a timer for detecting a logic level at an idle state;
a flip-flop having an output, the flip-flop changing logic states according to an external signal;
at least one logic gate for inverting said external signal.

2. A communication apparatus as claimed in claim 1, wherein an input of at least one logic gate is connected to said output of the flip-flop so as to invert said external signal or not invert said external signal according to the logic state of said flip-flop.

3. A communication apparatus as claimed in claim 1 or claim 2 configured to receive said external signal from a recieve input of said interchange circuit.

4. A communication apparatus as claimed in any one of claims 1-3, comprising an input terminal connected directly to said flip-flop.

5. A communication apparatus as claimed in any one of claims 1, 2 or 3, wherein said flip-flop is connected to said timer.

6. A communication apparatus as claimed in claim 5, wherein a trigger of said flip-flop is connected to an output of said timer.

7. A communication apparatus as claimed in any one of claims 1-6, comprising a first input terminal and a second input terminal for detecting a logic level at respective receiving pins.

8. A communication apparatus as claimed in any one of claims 1-7, wherein said logic level is high at said idle state when said input terminals are correctly connected.

9. A communication apparatus as claimed in any one of claims 1-8, wherein said logic level is low during said idle state when said input terminals are wrongly connected.

10. A communication apparatus as claimed in any one of claims 1-9, wherein said timer resets and generates a negative going pulse from said timer output when said input terminals are correctly connected.

11. A communication apparatus as claimed in any one of claims 1-10, wherein said timer will start counting and generates a logic low signal with a negative going pulse from said timer output when said input terminals are wrongly connected.

12. A communication apparatus as claimed in claim 1, wherein said flip-flop is triggered by said pulse from said timer output.

13. A communication apparatus as claimed in any of claims 1 to 12, wherein said at least logic gate comprises a pair of XOR gates.

14. A communication apparatus as claimed in claim 13, wherein an output of the flip-flop is connected to said XOR gates.

15. A communication apparatus as claimed in claim 13, where outputs of the XOR gates are at logic low level at the idle state when input terminals of said interchange circuit are correctly connected.

16. A communication apparatus as claimed in any one of claims 13-15, wherein the output of said XOR gates are operable to invert the signal logic level to a high logic during the idle state when input terminals of said interchange circuit are wrongly connected so that the connection system will be able to interpret a receive signal to establish communications between differential interchange circuit and multiple devices.

17. A communication apparatus as claimed in any one of claims 1 to 16, wherein the interchange circuit has input and output terminals, and wherein the communication apparatus is adapted for detecting an error in the connection of an interchange circuit with multiple devices and converting a signal at input or output terminals of the interchange circuit.

18. A communication apparatus as claimed in any one of claims 1 to 17 for use with a differential interchange circuit wherein input and output terminals have correct and wrong connection configurations with respect to other differential signals devices.

19. A communication apparatus for detecting the error in the signal and converting signal between a differential interchange circuit having input and output terminals:
a timer for detecting logic level at idle state;
a flip-flop having at least one output terminal for switching a signal from one to the other by means of an external signal; and
at least one or more of logic gates for inverting said external signal from said circuit.

20. A method for converting a signal between multiple devices and a differential interchange circuit, the method implemented by a communication apparatus, the method including:
receiving signals from the differential interchange circuit;
detecting a logic level at a receiving input terminal of the differential interchange circuit;
resetting via the logic level a timer in the communication apparatus and generating a pulse from an output of the timer;
triggering a flip-flop in the communication apparatus;
inverting the signals received; and
interpreting the received signals and establishing communication with the connected devices.
